# EUROPÄISCHE PATENTSCHRIFT

(11) **EP 2 344 138 B1**
(45) Veröffentlichungstag und Bekanntmachung des Hinweises auf die Patenterteilung: **16.07.2014**
(21) Anmeldenummer: 09783239.8
(22) Anmeldetag: 21.09.2009
(51) Int. Cl.: A61K 47/34, A61K 9/16, A61K 9/20

(54) **VERWENDUNG VON COPOLYMEREN AUF BASIS VON POLYETHERN UND VINYLMONOMEREN ALS BINDEMITTEL FÜR FESTE WIRKSTOFFHALTIGE DOSIERUNGSFORMEN**
USE OF POLYETHER-BASED AND VINYL MONOMER-BASED COPOLYMERS AS BINDERS FOR DOSING FORMS COMPRISING SOLID ACTIVE INGREDIENTS
UTILISATION DE COPOLYMÈRES À BASE DE POLYÉTHERS ET DE MONOMÈRES VINYLIQUES EN TANT QUE LIANTS POUR DES FORMES POSOLOGIQUES SOLIDES CONTENANTS DES PRINCIPES ACTIFS

(30) Priorität: 25.09.2008 EP 08165113
(43) Veröffentlichungstag der Anmeldung: 20.07.2011
(73) Patentinhaber: BASF SE, 67056 Ludwigshafen (DE)
(72) Erfinder: MEYER-BÖHM, Kathrin, 90537 Feucht (DE); DOBRAWA, Rainer, 70469 Stuttgart (DE); MASCHKE, Angelika, 93047 Regensburg (DE); KOLTER, Karl, 67117 Limburgerhof (DE)
(86) Internationale Anmeldenummer: PCT/EP2009/062199
(87) Internationale Veröffentlichungsnummer: WO 2010/034688

(56) Entgegenhaltungen:
- WO-A2-2007/051743
- DE-A1- 2 016 470
- DE-A1- 19 844 903

## Beschreibung

Die vorliegende Erfindung betrifft die Verwendung von Copolymeren, die durch Polymerisation von Vinylacetat und N-Vinyllactamen in Gegenwart eines Polyethers erhalten werden, als Bindemittel, insbesondere alsTrockenbindemittel, für feste wirkstoffhaltige Dosierungsformen, insbesondere Tabletten.

Bindemittel verbessern die Verpressbarkeit von wirkstoffhaltigen Formulierungen zu festen Dosierungsformen durch Verbesserung der Partikelhaftung.

Kriterien des Qualitätsstandards bei der Prüfung von komprimierten Dosierungsformen wie den Tabletten sind gemäß dem Europäischen Arzneibuch Gleichförmigkeit der Masse (Gewichtseinheitlichkeit), Bruchfestigkeit, Friabilität und Zerfall.

Cellulosepulver, zum Beispiel mikrokristalline oder mikrofeine Cellulosen, führen zu sehr harten Tabletten, beeinträchtigen aber die Fliessfähigkeit der Tablettiermischung.

Weitere Bindemittel wie Hydroxypropylmethylcellulosen oder Polyvinylpyrrolidone stellen sehr effektive Feuchtbindemittel dar.

Zu den Trockenbindemitteln für die Granulation ohne Zusatz von Lösungmitteln oder die Direktverpressung kommen Hydroxypropylcellulosen oder Copovidon (Copolymer aus N-Vinylpyrrolidon und Vinylacetat im Gewichtsverhältnis 60:40) in Betracht.

Häufig vermögen die bisher bekannten Bindemittel, insbesondere solche, die als Trockenbindemittel geeignet sind, den verschiedenen Kriterien für die Qualittätsstandards nur teilweise zu genügen. So verhalten sich Tablettenfestigkeit und Zerfallszeit oft wie Antipoden. Auch die Friabilität, also der Abrieb, kann sowohl bei weichen Tabletten durch Kantenabrieb als auch bei sehr harten Tabletten durch Deckeltendenzen unbefriedigend hoch sein.

Es bestand daher die Aufgabe, neue und verbesserte Bindemittel, insbesondere Trockenbindemittel, für pharmazeutische, kosmetische, lebensmitteltechnische, agrotechnische oder sonstige technische Anwendungen bereitzustellen, die hinsichtlich Bruchfestigkeit und Friabilität der Dosierungsformen verbesserte Eigenschaften aufweisen.

Aus der WO 2007/051743 ist die Verwendung von Copolymerisaten, die durch Polymerisation von N-Vinyllactam, Vinylacetat und Polyethern erhalten werden, als Löslichkeitsvermittler für in Wasser schwerlösliche Wirkstoffe bekannt.

Das Dokument DE 198 44 903 offenbart die Verwendung von Polymerisaten als Bindemittel, die erhältlich sind durch Polymerisation von a) mindestens einem Vinylester von aliphatischen C1-C24-Carbonsäuren, in Gegenwart von b) Polyethern und anschließender vollständiger oder teilweiser Verseifung der Polyvinylestergruppen, ferner möglicherweise einem weiteren Monomer c) wie z.B. N-Vinyllactame.

Die Aufgabe wurde erfindungsgemäß gelöst durch die Verwendung von wasserlöslichen oder wasserdispergierbaren Copolymerisaten, die erhalten werden durch radikalisch initiierte Polymerisation einer Mischung aus
i) 40 bis 60 Gew.-% N-Vinylcaprolactam
ii) 15 bis 35 Gew.- % Vinylacetat
iii) 10 bis 30 Gew.-% eines Polyethylenglykols mit einem Molekulargewicht von 1500 bis 10.000 Dalton,
   mit der Maßgabe, dass die Summe von i), ii) und iii) gleich 100 Gew.-% ist, als Bindemittel für die Herstellung von festen wirkstoffhaltigen Dosierungsformen.

Die Molekulargewichte werden ausgehend von der gemäß DIN 53240 gemessenen OH-Zahl bestimmt.

Allgemeine Verfahren zur Herstellung der erfindungsgemäßen Copolymerisate sind an sich bekannt. Die Herstellung erfolgt durch radikalisch initiierte Polymerisation, bevorzugt Lösungspolymerisation, in nichtwässrigen, organischen Lösungsmitteln oder in gemischt nichtwässrigen/wässrigen Lösungsmitteln. Die Herstellung der Copolymerisate und deren Überführung in die Pulverform ist in der WO 2007/051743, auf deren Offenbarung hinsichtlich der Herstellung der Copolymerisate hiermit ausdrücklich Bezug genommen wird, ausführlich beschrieben.

Die Polymerisate weisen K-Werte nach Fikentscher im Bereich von 10 bis 60, vorzugsweise 15 bis 40, gemessen in einer 1 gew.-%igen ethanolischen Lösung, auf.

Die Polymerisate eignen sich erfindungsgemäß als Bindemittel zur Herstellung von festen Dosierungsformen, insbesondere als Bindemittel für die Herstellung von Tabletten.

Die Polymerisate weisen Glasübergangstemperturen im Bereich von 30 bis 120, bevorzugt von 50 bis 80 °C auf.

Bevorzugt ist die Verwendung als Trockenbindemittel für Pulvermischungen zur Direkttablettierung.

Die Polymerisate eignen sich aber auch zum Einsatz in der Feuchtgranulierung, Wirbelschichtgranulierung oder Trockengranulierung.

Bei der Feuchtgranulierung kann das Bindemittel in Form einer wässrigen oder auch organischen Lösung die vorzugsweise 1 bis 30 Gew.-% Bindemittel enthält, eingesetzt werden.Die Polymerisatlösung kann beispielsweise in einem Mischer oder Kneter mit den weiteren Einsatzstoffen granuliert werden.

Bei der Wirbelbettgranulation kann das Bindemittel in Form einer Lösung, bevorzugt einer wässrigen Lösung, auf ein Wirbelbett von pulverförmigen Komponenten gesprüht werden, wobei es zu einer Agglomeration kommt. Im Wirbelbett können sich die pharmazeutischen Wirkstoffe und/oder weitere pharmazeutische Hilfsstoffe befinden.

Gemäß einer weiteren bevorzugten Ausführungsform eignen sich die Polymerisate auch zur Verarbeitung durch Sintergranulierung. Dabei werden die Polymerisate durch den Energieeintrag, der durch den Mischvorgang erfolgt, und/oder durch erhöhte Temperatur zum Erweichen gebracht, wodurch es zu einer Agglomeration der eingesetzten Komponenten kommt. Durch das Erweichen und spätere Erstarren nach Abkühlung kommt es zur Ausbildung von Feststoffbrücken. Dabei kann der Prozess so geführt werden, dass es zu einem oberflächlichen oder teilweisen Erweichen der Polymerisatpartikel kommt. Es ist auch möglich, die Sintergranulation so zu betreiben, dass es zu einer vollständigen Erweichung der Polymerisate kommt. Ein vollständiges Erweichen kann sich empfehlen, wenn besonders starke Bindemittelbrücken erwünscht sind.

Die Sintergranulation kann in einem beheizbaren Mischer, Kneter oder einem Schneckenextruder erfolgen.

Bei Verarbeitung in einem Schneckenextruder wird der Extruder ohne Düsenplatte, also in offener Fahrweise, verwendet. Das Verfahren erfolgt drucklos. Durch geeignete Wahl der Schneckenkonfiguration kann die Partikelgröße der Granulate mit einer engen Partikelgrößenverteilung eingestellt werden. Wie die Schneckenkonfiguration in Abhängigkeit von der eingesetzten Mischung für diesen Zweck gewählt werden soll, kann der Fachmann durch einige geeignete Versuche ermitteln.

Die Sintergranulation kann bei Temperaturen von 50 bis 120, bevorzugt 70 bis 100 °C, erfolgen. Welche Temperatur besonders geeignet ist, hängt von der Zusammensetzung der Mischung ab. Bei Mitverarbeitung von weichmachenden Substanzen, können gegebenenfalls niedrigere Temperaturen gewählt werden.

Unabhängig davon, welche Granulierungsmethode verwendet wird, kann man beispielsweise die pharmazeutischen Wirkstoffe sowie weitere pharmazeutische Hilfsstoffe wie grenzflächenaktive Verbindungen, weitere Bindemittel, Füllstoffe, Säuerungsmittel, Puffersubstanzen, Aromastoffe, Stabilisierungsmittel oder farbgebende Substanzen in die Granulate einarbeiten.

Der pharmazeutische Wirkstoff kann zur Herstellung der festen Dosierungsformen intragranulär oder extragranulär verarbeitet werden. So ist es möglich, Granulate, die weitere pharmazeutische Hilfsstoffe enthalten, jedoch keinen Wirkstoff, herzustellen und diese dann mit den Wirkstoffen zu vermischen. Es ist auch möglich, einen Wirkstoff sowohl intragranulär und extragranulär einzuarbeiten. Es können auch Kombinationen unterschiedlicher Wirkstoffe eingesetzt werden. Bei miteinander unverträglichen Wirkstoffen kann der eine intragranulär und der andere extragranulär verarbeitet werden.

Die Granulatteilchen können mittlere Teilchengrößen von 100 bis 1000µm, bevorzugt 200 bis 600 µm aufweisen. In den Granulatteilchen kann der Anteil an erfindungsgemäßem Bindemittel 1 bis 60, bevorzugt 5 bis 30 gew.-% betragen.

Das erfindungsgemäße Bindemittel kann in Mengen von 1 bis 20 Gew.-%, bevorzugt 1 bis 10 Gew.-%, bezogen auf das Gesamtgewicht der Dosierungsform, eingesetzt werden.

Pharmazeutische Zubereitungen zur Herstellung von festen Dosierungsformen können neben den erfindungsgemäßen Bindemitteln auch übliche pharmazeutische Hilfsstoffe wie Füllstoffe, Sprengmittel, farbgebende Mittel, Aromen, filmbildende wasserunlösliche Polymere, Verdickungsmittel oder grenzflächenaktive Mittel sowie weitere übliche Bindemittel in den hierfür üblichen Mengen enthalten.

Als Füllstoffe eignen sich beispielsweise Zucker, Zuckeralkohole oder Mischungen davon. Als Zucker oder Zuckeralkohole eignen sich Trehalose, Mannit, Erythrit, Isomalt, Maltit, Lactit, Xylit, Sorbit. Ferner können auch anorganische Substanzen als Füllstoffe verwendet werden. Hier eignen sich Di- und Tricalciumphosphate.

Als Sprengmittel können in Mengen von 1 bis 25 Gew.-%, bevorzugt, 2 bis 15 Gew.-%, besonders bevorzugt 3 bis 10 Gew.-%, eingesetzt werden. Solche Sprengmittel sind wasserunlöslich, aber nicht filmbildend. Als Sprengmittel eignet sich beispielsweise Crospovidon, ein quervernetzes Polymerisat aus N-Vinylpyrrolidon. Weiterhin eignet sich Croscarmellose, eine quervernetzte Carboxymethyllcellulose, wobei als Croscarmellose erfindungsgemäß auch deren Natrium- und Calciumsalze gemeint sind. Weiterhin eignet sich Natriumcarboxymethylstärke. Ebenso eignet sich L-Hydroxypropylcellulose, bevorzugt mit 5 bis 16% Hydroxypropyl-Gruppen.

Als weitere Hilfsstoffe können wasserunlösliche Polymere, die im pH-Bereich von 1 bis 14 unlöslich sind, also eine bei jedem pH-Wert pH-unabhängige Wasserunlöslichkeit aufweisen, eingesetzt werden. Weiterhin eignen sich aber auch Polymere, die bei jedem pH-Wert im pH-Bereich von 6 bis 14 wasserunlöslich sind, in saurem Milieu aber löslich sind. Die Polymere können filmbildende Polymere sein. Filmbildend bedeutet in diesem Zusammenhang, dass die Polymere in wässriger Dispersion eine Mindestfilmbildetemperatur von -20 bis +150 °C, bevorzugt 0 bis 100 °C aufweisen. Geeignete Polymere sind Polyvinylacetat, Ethylcellulose, Methylmethacrylat-Ethylacrylat-Copolymere, Ethylacrylat-Methylmethacrylat-Trimethylammoniumethylmethacrylat-Terpolymere. Butylmethacrylat-Methylmethacrylat-Dimethylaminoethylmethacrylat-Terpolymere

Die Acrylat-Methacrylat-Copolymere sind näher beschrieben in der Europäischen Pharmacopoeia als Polyacrylate Dispersion 30%, in der USP als Ammonio Methacrylate Copolymer und in JPE als Aminoalkyl-Methacrylate Copolymer E.

Polyvinylacetat kann als wässrige Dispersion mit Feststoffgehalten von 10 bis 45 Gew.-% eingesetzt werden. Bevorzugt ist zudem Polyvinylacetat mit einem Molekulargewicht zwischen 100.000 und 1.000.000 Dalton besonders bevorzugt zwischen 200.000 und 800.000 Dalton.

Weiterhin können die Formulierungen als Komponenten zusätzliche wasserlösliche Polymere in Mengen von 0 bis 15 Gew.-% enthalten. Geeignete wasserlösliche Polymere sind beispielsweise Polyvinylpyrrolidone, Vinylpyrrolidon-Vinylacetat-Copolymere, Polyvinylalkohole, Polyvinylalkohol-Polyethylenglykol-Pfropfcopolymere, Polyethylenglykole oder Ethylenglykol-Propylenglykol-Blockcopolymere.

Weiterhin können Verdickungsmittel wie Methylcellulose, Hydroxypropylmethylcellulose, Hydroxypropylcellulose, Hydroxyethylcellulose, Carragenane, Pektine, Xanthane oder Alginate eingesetzt werden. Durch Zusatz von Verdickungsmitteln wie beispielsweise hochmolekularen Polysacchariden kann das Mundgefühl durch Erhöhung der Weichheit und des Volumengefühls zusätzlich verbessert werden.

Gewünschtenfalls können weitere pharmazeutisch üblichen Hilfsstoffe in Mengen von 0 bis 15 Gew.-%, beispielsweise wie Säuerungsmittel, Puffersubstanzen, Süßstoffe, Aromen, Geschmacksverstärker und Farbstoffe eingesetzt werden. Folgende Stoffe sind hierbei besonders geeignet: Citronensäure, Weinsäure, Ascorbinsäure, Natriumdihydrogenphosphat, Cyclamat, Saccharin-Na, Aspartam, Menthol, Pfefferminzaroma, Fruchtaromen, Vanillearoma, Glutamat, Riboflavin, Betacarotin, wasserlösliche Farbstoffe, feinteilige Farblacke.

Weiterhin können auch grenzflächenaktive Verbindungen zugegeben werden, beispielsweise Natriumlaurylsulfat, Dioctylsulfosuccinat, alkoxilierte Sorbitanester wie Polysorbat 80, polyalkoxilierte Derivate von Rizinusöl oder hydriertem Rizinusöl, beispielsweise Cremophor® RH 40, alkoxilierte Fettsäuren, alkoxilierte Hydroxyfettsäuren, alkoxilierte Fettalkohole, Alkalisalze von Fettsäuren und Lecithine.

Weiterhin können auch Pigmente wie Eisenoxide, Titandioxid, kolloidale oder gefällte Kieselsäure, Calciumcarbonate oder Calciumphosphate zugegeben werden.

Die fertigen Tabletten können auch mit den üblichen Überzügen versehen werden.

### Beispiele

Die Bestimmung der Böschungswinkel und der Fließgeschwindigkeit erfolgte nach DIN ISO 4324. Die Schüttdichte wurde gemäß Pharm. Eur. 5 bestimmt.

Verwendete Geräte: Tablettentester, (Krämer)Tablettenzerfallstester ZT 74 (ERWEKA) Friabilitätstester TAR 20 (ERWEKA)

### Beispiel 1: Trockenbindemittel

Die Beurteilung der Bindekraft erfolgte an einem Polymerisat A, erhalten aus 13 Gew.-% Polyethylenglykol PEG 6000, 57 Gew.-% N-Vinylcaprolactam und 30 Gew.-% Vinylacetat mit einem K-Wert von 35.

Zum Vergleich wurde die Bindekraft von Povidon K30 (Polyvinylpyrrolidon K-Wert 30) und von Copovidon (Copolymer aus N-Vinylpyrrolidon und Vinylacetat im Gewichtsverhältnis 60:40, K-Wert 28; Kollidon® VA 64, Fa. BASF) sowie von Mischungen ohne Bindemittel wie folgt beurteilt.

| Beurteilung | gut | mittel | schlecht |
|---|---|---|---|
| Bruchfestigkeit [N] | > 75 | 60-75 | < 60 |
| Friabilität [%] | < 0.5 | 0.5-1.0 | > 1.0 |
| Zerfall [min] | < 4 | 4-6 | >6 |
| Gewichtseinheitlichkeit [%] | <0.6 | 0.6-0.8 | >0.8 |

Es wurden folgende Zusammensetzungen verwendet:
pro 500 mg Tablettengewicht
200,00 mg Ascorbinsäure-Pulver
231,25 mg Ludipress¹⁾
50,00 mg Bindemittel
15,00 mg Crospovidon²⁾
1,25 mg hochdisperse Kieselsäure³⁾
2,50 mg Magnesiumstearat
1) Ludipress®: Formulierung aus 93 Gew.-% Lactose, 3.5. Gew.-% Povidon K30 (Kollidon® 30, Firma BASF), 3.5 Gew.-% Crospovidon
2) Crospovidon: Kollidon® CL, Firma BASF
3) Aeosil® 200, Firma Evonik

Die Mischung der Komponenten wurde durch ein Sieb mit einer Maschenweite von 0.8 mm gegeben, in eine Glasflasche gefüllt und 10 Minuten in einem Turbula-Mischer gemischt und zu Tabletten verpresst.
Tablettenformat: 12 mm Durchmesser, facettiert
Presskraft: 18 kN
Tablettiergeschwindigkeit: 30 U/min Rundläuferpresse

Die Tabeltten wurden anschließend auf ihre physikalischen Eigenschaften überprüft.

| Bindemittel | Povidon K30 | Copovidon | ohne Bindemittel | Polymerisat A erfindungsgemäß |
|---|---|---|---|---|
| Bruchfestigkeit [N] | 65 | 90 | 49 | 87 |
| Friabilität [%] | 0.43 | 1.14 | 3.5(15% Bruch) | 0.44 |
| Zerfall [min.sec] | 1.19 | 1.02 | 0.24 | 2.30 |
| Gewichtseinheitlichkeit[%] | 4.12 | 0.58 | 1.01 | 0.34 |

Das erfindungsgemäß verwendete Bindemittel führt damit zu Tablettenbruchfestigkeiten, die vergleichbar mit denen von Copovidon enthaltenden Tabletten sind, gleichzeitig aber vor allem zu deutlich niedrigerer Friabilität.

### Beispiel 2: Feuchtbindemittel

| Rezeptur | Einwaage [g] | Zusammensetzung nach Trocknung [Gew.-%] |
|---|---|---|
| Calciumhydrogenphosphat | 350,00 | 80,8 |
| Lactose | 50,00 | 11,5 |
| Bindemittel | 12,40 | 2,9 |
| VE-Wasser | 55,00 | - |
| Kollidon CL | 18,56 | 4,3 |
| Magnesiumstearat | 2,06 | 0,5 |

Calciumhydrogenphosphat und Lactose wurden in einen Diosna-Mischer (Diosna P 1/6 (2 L Rührbehälter))eingewogen und die Mischung 1 min gemischt (200 rpm Rührer / 2200 rpm Zerhacker). Eine 18,4 gew.-%-ige wässrige Bindemittellösung wurde innerhalb von 4 min tropfenweise unter ständigem Rühren hinzugegeben (bei 200 rpm Rührer / 2200 rpm Zerhacker). Nach Zugabe der Bindemittellösung zur Mischung wurde für weitere 2 min gemischt. Anschließend wurde die Rührerumdrehung für 30 s auf 1500 rpm erhöht, der Zerhacker lief weiterhin mit 2200 rpm. Danach wurde die befeuchtete Masse durch ein Sieb der Maschenweite 0,8 mm gegeben und das feuchte Granulat 12 Stunden an der Luft auf Horden getrocknet. Der absolute Trocknungsverlust des Granulats wurde mit Hilfe eines IR - Trockners bis zur Massenkonstanz bei 80 °C bestimmt. Anschließend wurden Kollidon CL und Magnesiumstearat zugegeben. Die gesamte Masse wurde durch ein Sieb der Maschenweite 0,8 mm gegeben, in eine Glasflasche gefüllt, verschlossen und 5 min im Turbula - Mischer (Turbula Mischer T2, Willy A Bachofen AG)gemischt.

Anschließend wurden wie unter Beispiel 1 beschrieben Tabletten hergestellt und getestet.

**Tablettenanalyse:**

| Pressdruck | Bindemittel | Bruchfestigkeit [N] | Friabilität [%] | Zerfall [s] |
|---|---|---|---|---|
| 18kN | Erfindungsgemäß, Polymerisat A | 103 | 0.13 | 31 |
| | Kollidon VA 64 | 83 | 0.37 | 38 |
| | Kollidon 30 | 95 | 0.18 | 35 |
| | Kollidon 90 F | 109 | 0.15 | 117 |

Das erfindungsgemäße verwendete Polymerisat wies im Vergleich mit bekannten Bindemitteln eine hohe Bindekraft bei gleichzeitig verbesserter Friabilität und schnellerem Zerfall auf.

### Beispiel 3: Sintergranulat

| Rezeptur | Zusammensetzung [Gew.- %] |
|---|---|
| Polymerisat A | 50 |
| Lactose | 45 |
| PEG 400 | 5 |

Das Polymerisat und die Lactose (Granulac® 230, mittlere Teilchengröße 27 µm) wurden in einen Stephan- Mischer (UMC 5) eingewogen. Der Produktbehälter wurde mittels doppelwandiger Ausführung und angeschlossenem Ölbad auf 95°C temperiert. Diese Mischung wurde 5 Minuten gemischt (bei 1000 UpM Rührergeschwindigkeit). Nach Erreichen einer homogenen Mischung wurde tropfenweise Polyethylengykol (PEG 400 ; Lutrol® E 400, BASF SE) zu der Mischung hinzugegeben und bei gleicher Umdrehungszahl für weitere 20 Minuten weitergemischt. Das fertige Granulat wurde im Anschluss durch ein Sieb (Maschenweite 1000µm) gesiebt, um störenden Grobkornanteil zu entfernen.

Die Charakterisierung des resultierenden Granulats zeigte eine erfolgreiche Agglomeration der Lactose. Der mittlere Partikeldurchmesser vergrößerte sich von 27µm (Granulac 230) auf 396 µm (Lactosegranulat mit Polymerisat A).
Überraschenderweise wies das entstandene Granulat kaum Feinanteil auf, was für ein gleichmäßiges Agglomerationsverhalten spricht. Dies wird durch den d₁₀-Wert von 228 µm belegt, was bedeutet, dass weniger als 10 Gew.-% der Teilchen eine teilchengröße von kleiner 228 µm aufweisen.

Die Schüttdichte des Granulats betrug bei 0,63 g/mL bestimmt mit dem Schütt- / Stampfvolumeter nach Ph.Eur. Das Fließverhalten des Granulats bestimmt mittels Pfrengle - Trichter wies einen Böschungswinkel von 26,6° und eine Auslaufzeit von 7,2 Sekunden auf.

Anschließend wurde das Granulat zu Tabletten verpresst. Vor der Tablettierung des fertigen Granulats wurden 5 Gew.-% Sprengmittel (Crospovidone; Kollidon® CL) und 0,5 Gew.-% Schmiermittel (Magnesiumstearat) zugegeben.. Die gesamte Masse wurde erneut durch ein Sieb der Maschenweite 1,0 mm gegeben, in eine Glasflasche gefüllt, verschlossen und 5 min im Turbula -Mischer gemischt. Die Pressmischung wurde auf einer Exzenterpresse (Exzenterpresse EK0) mit 18kN zu 12mm großen Tabletten verpresst.

Die Tabletten wiesen folgende Eigenschaften auf:
Bruchfestigkeit: 80 N
Friabilität: 0.2 %
Zerfall: 50 s

### Beispiel 4: Sintergranulate; Prozentangaben [Gew.-%]

| Formulierung 1 | Formulierung 2 | Formulierung 3 | Formulierung 4 |
|---|---|---|---|
| 50 % Polymerisat A | 50 % Polymerisat A | 50 % Polymerisat A | 40 % Polymerisat A |
| 30 % Lactose | 30 % Lactose | 25 % Lactose | 35 % Lactose |
| 20 % Fenofibrat | 15 % Itraconazol | 20 % Cinnarizin | 20 % Cinnarizin |
| - | 5 % PEG 400 | PEG 5 % 600 | 5 % PEG 600 |

Die Herstellung der Granulate erfolgte in einem Zweischneckenextruder mit 16 mm Schneckendurchmesser (L/D Verhältnis = 40) der Firma ThermoFisher Polylab, der drucklos mit offenem Kopf betrieben wurde. Granuliert wurde bei 100 rpm Schneckendrehzahl und einer Zylindertemperatur (ab dem 2. Zylinderelement) von 80°C.

| | Formulierung 1 | Formulierung 2 | Formulierung 3 | Formulierung 4 |
|---|---|---|---|---|
| Böschungswinkel | 28 ° | 30 ° | 30 ° | 31 ° |
| Fließgeschwindigkeit | 8 sec | 7 sec | 8.1 sec | 7.5 sec |
| Schüttdichte | 0.6 g/ml | 0.65 g/ml | 0.55 g/ml | 0.58 g/ml |
| Mittlerer Partikeldurchmesser | 350 µm | 420 µm | 342 µm | 380 µm |

## Patentansprüche

1. Verwendung von wasserlöslichen oder wasserdispergierbaren Copolymerisaten, die erhalten werden durch radikalisch initiierte Polymerisation einer Mischung aus
i) 40 bis 60 Gew.-% N-Vinylcaprolactam
ii) 15 bis 35 Gew.-% Vinylacetat
iii) 10 bis 30 Gew.-% eines Polyethylenglykols mit einem Molekulargewicht von 1500 bis 10.000 Dalton,
mit der Maßgabe, dass die Summe von i), ii) und iii) gleich 100 Gew.-% ist,
als Bindemittel für die Herstellung von festen wirkstoffhaltigen Dosierungsformen.

2. Verwendung nach Anspruch 1, zur Herstellung von Tabletten.

3. Verwendung nach Anspruch 1 oder 2, wobei das Bindemittel in Mengen von 1 bis 20 Gew.-%, bezogen auf das Gesamtgewicht der Dosierungsform, eingesetzt wird.

4. Verwendung nach einem der Ansprüche 1 bis 3, wobei das Bindemittel in Mengen von 1 bis 10 Gew.-% in den Dosierungsformen eingesetzt wird.

5. Verwendung nach einem der Ansprüche 1 bis 4, wobei die Bindemittel als Trockenbindemittel eingesetzt werden.

6. Verwendung nach einem der Ansprüche 1 bis 5,wobei die Bindemittel als Trockenbindemittel für die Direkttablettierung eingesetzt werden.

7. Verwendung nach einem der Ansprüche 1 bis 4, wobei die Bindemittel in Form von Feuchtgranulaten oder Wirbelschichtgranulaten eingesetzt werden.

8. Verwendung nach einem der Ansprüche 1 bis 4, wobei die Bindemittel in Form von Sintergranulaten eingesetzt werden.

9. Verwendung nach Anspruch 8, wobei die Sintergranulate bei Temperaturen von 50 bis 100 °C erhalten werden.

10. Verwendung nach Anspruch 8, wobei die Sintergranulate bei Temperaturen von 70 bis 100 °C erhalten werden.

11. Verwendung nach einem der Ansprüche 1 bis 4 und 7 bis10, wobei die Bindemittel in Gegenwart von weiteren pharmazeutischen Hilfsstoffen granuliert werden.

12. Verwendung nach einem der Ansprüche 1 bis 4 und 7 bis11, wobei die Bindemittel in Gegenwart von pharmazeutischen Wirkstoffen granuliert werden.

## Claims

1. The use of water-soluble or water-dispersible copolymers which are obtained by free radical-initiated polymerization of a mixture of
i) 40 to 60% by weight of N-vinylcaprolactam
ii) 15 to 35% by weight of vinyl acetate
iii) 10 to 30% by weight of a polyethylene glycol having a molecular weight of from 1500 to 10,000 daltons,
with the proviso that the total of i), ii) and iii) is equal to 100% by weight,
as binders for producing solid active ingredient-containing dosage forms.

2. The use according to claim 1, for producing tablets.

3. The use according to claim 1 or 2, where the binder is employed in amounts of from 1 to 20% by weight based on the total weight of the dosage form.

4. The use according to any of claims 1 to 3, where the binder is employed in amounts of from 1 to 10% by weight in the dosage forms.

5. The use according to any of claims 1 to 4, where the binders are employed as dry binders.

6. The use according to any of claims 1 to 5, where the binders are employed as dry binders for direct tableting.

7. The use according to any of claims 1 to 4, where the binders are employed in the form of wet granules or fluidized-bed granules.

8. The use according to any of claims 1 to 4, where the binders are employed in the form of sintered granules.

9. The use according to claim 8, where the sintered granules are obtained at temperatures of 50 to 100°C.

10. The use according to claim 8, where the sintered granules are obtained at temperatures of 70 to 100°C.

11. The use according to any of claims 1 to 4 and 7 to 10, where the binders are granulated in the presence of further pharmaceutical excipients.

12. The use according to any of claims 1 to 4 and 7 to 11, where the binders are granulated in the presence of active pharmaceutical ingredients.

## Revendications

1. Utilisation de copolymères solubles ou dispersibles dans l'eau, qui sont obtenus par polymérisation initiée par voie radicalaire d'un mélange constitué par
i) 40 à 60% en poids de N-vinylcaprolactame
ii) 15 à 35% en poids d'acétate de vinyle,
iii) 10 à 30% en poids d'un polyéthylèneglycol présentant un poids moléculaire de 1500 à 10 000 Daltons,
sous réserve que la somme de i), ii) et iii) soit égale à 100% en poids, comme liant pour la préparation de formes de dosage solides contenant des substances actives.

2. Utilisation selon la revendication 1 pour la fabrication de comprimés.

3. Utilisation selon la revendication 1 ou 2, le liant étant utilisé en des quantités de 1 à 20% en poids, par rapport au poids total de la forme de dosage.

4. Utilisation selon l'une quelconque des revendications 1 à 3, le liant étant utilisé en des quantités de 1 à 10% en poids dans les formes de dosage.

5. Utilisation selon l'une quelconque des revendications 1 à 4, les liants étant utilisés comme liants à sec.

6. Utilisation selon l'une quelconque des revendications 1 à 5, les liants étant utilisés comme liants à sec pour le pastillage direct.

7. Utilisation selon l'une quelconque des revendications 1 à 4, les liants étant utilisés sous forme de granulats humides ou de granulats en lit fluidisé.

8. Utilisation selon l'une quelconque des revendications 1 à 4, les liants étant utilisés sous forme de granulats frittés.

9. Utilisation selon la revendication 8, les granulats frittés étant obtenus à des températures de 50 à 100°C.

10. Utilisation selon la revendication 8, les granulats frittés étant obtenus à des températures de 70 à 100°C.

11. Utilisation selon l'une quelconque des revendications 1 à 4 et 7 à 10, les liants étant granulés en présence d'autres adjuvants pharmaceutiques.

12. Utilisation selon l'une quelconque des revendications 1 à 4 et 7 à 11, les liants étant granulés en présence de substances actives pharmaceutiques.
